**Europäisches Patentamt**

(19) **European Patent Office**

**Office européen des brevets**

(11) Publication number : **0 333 342 B1**

(12) # EUROPEAN PATENT SPECIFICATION

(45) Date of publication of patent specification :
10.03.93 Bulletin 93/10

(51) Int. Cl.⁵ : **A23L 1/308, A61K 35/78**

(21) Application number : **89301911.7**

(22) Date of filing : **27.02.89**

(54) **Use of the fruit of a plant of the genus Ocimum in the preparation of an obesity control agent.**

(30) Priority : **03.03.88 JP 50845/88**

(43) Date of publication of application :
**20.09.89 Bulletin 89/38**

(45) Publication of the grant of the patent :
**10.03.93 Bulletin 93/10**

(84) Designated Contracting States :
**AT BE CH DE ES FR GB GR IT LI LU NL SE**

(56) References cited :
**CHEMICAL ABSTRACTS, vol. 103, 1985, page 310, abstract no. 92700z, Columbus, Ohio, US; M.M. PATEL et al.: "Investigation in the properties of mucilage of oscimum canum sims. (Bavchi) as a pharmaceutical aid"**
**FOOD TECHNOLOGY, vol. 40, no. 2, February 1986, pages 104-110, Chicago, Illinois, US; B.O. SCHNEEMAN: "Physical and chemical properties, methods of analysis, and physiological effects"**
**CHEMICAL ABSTRACTS, vol. 108, 1988, page 422, abstract no. 118797s, Columbus, Ohio, US; M.M. PATEL et al.: "Mucilages of lepidium sativum, linn. [Asario] and ocimum canum, sims. [Bavchi] as emulsifiers"**

(73) Proprietor : **NIPPON SHINYAKU COMPANY, LIMITED**
**14, Kisshoin Nishinosho Monguchicho**
**Minami-ku Kyoto-shi Kyoto 601 (JP)**
Proprietor : **SIOE PHARMACEUTICAL COMPANY LTD.**
**2-5-7 Doshomachi Chuoku**
**Osaka 541 (JP)**

(72) Inventor : **Mitsuoka, Sachihiko**
**2-5-18 Sujaku**
**Nara-shi 631 (JP)**
Inventor : **Nishi, Toyoyuki**
**35 Nishimachi**
**Kameoka-shi Kyoto-fu 621 (JP)**
Inventor : **Yoshikuni, Yoshiaki**
**4-101 Uni Ujigawa Mansion 33 Kohata Nishinaka**
**Uji-shi Kyoto-fu 611 (JP)**
Inventor : **Fujita, Shunsaku**
**4-15-6 Mayumi Ikoma-shi**
**Nara-ken 630-01 (JP)**

(74) Representative : **Hillier, Peter et al**
**Reginald W. Barker & Co., 13, Charterhouse Square**
**London, EC1M 6BA (GB)**

## Description

The present invention relates to dietary compositions to assist in the reduction of obesity.

Obesity is mainly caused by excessive calorific intake, and many ways have been suggested for reducing the intake of food (dieting).

So-called 'health foods' have become popular in recent years. For example, glucomannan has had large sales as a so-called 'dietary fibre' product but has now lost popularity. Thus, whilst glucomannan is effective in inhibiting the adsorption of food in the intestines, it also has the disadvantage of decreasing appetite when taken during a meal. Accordingly it is difficult to take it over a long period of time.

Health foods known as 'dietary fibre' include not only glucomannan but suitable mixtures comprise vegetable fibre as the base pectin, konjacmannan, natural calcium and NDF, which are currently commercially available. It is said that these products have some effect in inhibiting the adsorption of food in the intestine. However, as medicants for the treatment of obesity, it is difficult to take such dietary fibre products over a sufficiently long period of time because the patients lose their appetites. (If it is not possible to take the product over a long period of time, obesity may temporarily be reduced but cannot be completely dealt with). Some of dietary fibre products cause adverse reactions in patients over long periods of administration.

If food compositions to be used for the control of obesity can be used only at each meal by adding them to food when cooked, they can easily be utilized in medical institutions. However, when such compositions are to be administered to a member of a family, it is often too troublesome to cook a special dish for the particular person every day.

Basically the present invention resides in the discovery that fruit of the Ocimum species may be used as active principle in obesity control agents or dietary control foodstuffs. Thus, the invention is concerned with the use of the fruit of a plant of the genus Ocimum in the preparation of an obesity control agent or dietary control foodstuff. The product may be orally administered to patients with food at each meal without affecting the taste or flavour of the food so as to improve obesity and satisfy the patients appetite after a meal.

The fruit of the plant of the genus Ocimum (hereinafter simply the 'Ocimum fruit') is most conveniently employed in dried form. Thus, the invention is particularly concerned with products using the dried fruit, which products may themselves be dried.

In the present invention there may be employed the fruit of any plants of the genus Ocimum, for example, typically, Ocimum canum Sims. (This is also called Kemeboki (in Japanese) belonging to the family Libiatae a tropical plant originally grown in Asia and Africa). Other Ocimum plants which may be used include Ocimum basilicum Linn, Ocimum sanctum Linn., Ocimum viride Willd., Ocimum graveolens A. Br., Ocimum micranthum Willd., and Ocimum kilimandscharicum Gurke.

The epicarp of the fruit of Ocimum canum swells in a jelly-like state when immersed in water and so it has been eaten as a dessert for a long time in South East Asia. However, this has not been used to treat obesity. The Ocimum fruit is known to improve the sugar tolerance of diabetics.

J.Med. Ass. Thailand Vo. 68, No. 8 (1985) describes that the daily oral administration of 30g of the fruit to a patient is effective in this regard.

The epicarp of the Ocimum fruit is found to be covered with a polysaccharide layer which swells to a jelly-like state when immersed in water.

We have found that Ocimum fruit contains NDF (Neutral Detergent Fibre such as hemicellulose, cellulose and lignin), ADF (Acid Detergent Fibre such as cellulose and lignin), fibre and sugar.

Whilst the effect of the Ocimum fruit might have been thought to depend only on the swelling of the conventional vegetable fibre in the digestive tract, we believe, as a result of the trials reported below, that the effect is not due to the same action as in the case of conventional dietary foods.

The product of the invention has three characteristic features, namely, (1) the product is suitable as a dieting agent because it satisfies the appetite for food for a long time or hardly causes the feeling of hunger; (2) the product can be orally administered over long periods of time because it gives no adverse reactions nor hardly decreases the appetite; and (3) the product can be preserved as a dry product and, if necessary, can be used immediately as an instant food. Conventional dietary foods does not have such characteristics.

Preferably the product is orally administered at a meal, preferably in a does of 1g - 100g (calculated of Ocimum fruit) a day, usually 10g a day. Although the term of administration can suitably be changed depending on the person to be treated and the degree of obesity, the product can usually be administered for a period of 3-6 months, for example about 4 months.

Food compositions in accordance with the invention can take the form of various dishes such as, preferably, Atsuyakitamago (eggroll), Sarasayaki, a Chinese dish of curry and fried rice, Mozuku (seaweed) seasoned with vinegar, meat and vegetables boiled in soy sauce, Hijiki (seaweed) boiled in soy sauce and sugar, and sherbet (kiwi fruit and orange); and more preferably, omelette and rice, bean curd, pancakes, hamburger

steaks, Chinese dishes dressed with sauce, eggplants boiled in thick soy with sugar, and Chikuzenni (vegetables and meat boiled in thick soy with sugar and oil).

A food composition of the invention can also suitably take the form of a dessert such as yoghurt, Fruit-mitsumame (fruit, agar-agar cubes and boiled beans dressed with syrup), Shiruko (azuki-bean soup with rice cakes), and snacks. In this case, the Ocimum fruit in accordance with the present invention is preferably incorporated in an amount of 1-6%. Sugar and colouring matter can be added, if necessary.

The dry food composition of the invention can be stored for a long time and eaten immediately after the addition of water at any time. The dry composition of the invention prepared by watering and then drying can be eaten instantly after the addition of water and so can serve as an instant food.

The dry food composition of the invention is found to be efficacious even when eaten at any time before, after or between meals.

Examples

The following are ingredient recipes for food compositions of the invention.

In the following examples, Ocimum canum Sims was used after having been dried, heated and sterilized (at 80°C for 3 hours) and is referred to in the Examples as the 'claimed product'.

Example 1

Claimed product    5g
Water              70g

Example 2

Kantenyose (Agar-agar-jelly)

| | |
|---|---|
| Claimed product | 2g |
| Agar-agar | 1g |
| Water | 20g |
| Marvie (trade mark - sweetening) | 15g |

Example 3

Temaki (vinegared agar-agar)

| | |
|---|---|
| Claimed product | 4g |
| Agar-agar | 2g |
| Water | 40g |
| Ohba | |
| (leaves of Perilla frutescens var. crispa) | 6 leaves |
| Soy sauce | 6 ml |
| Wasabi (Japanese horseradish) | a little |

3

Example 4

### Fruit-agar-agar jelly

| | |
|---|---|
| Claimed product | 4g |
| Agar-agar | 2g |
| Water | 20g |
| Kiwi fruit | 75g |
| Marvie (trade mark) | 30g |

Example 5

### Orange cup

| | |
|---|---|
| Claimed product | 4g |
| Agar-agar | 2g |
| Water | 20g |
| Orange | 100g |
| Marvie (trade mark) | 30g |

Example 6

### Flower cup

| | |
|---|---|
| Claimed product | 4g |
| Agar-agar | 2g |
| Water | 20g |
| Tangerine | 100g |
| Marvie (trade mark) | 30g |

Example 7

### Fruit cup

| | |
|---|---|
| Claimed product | 2g |
| Agar-agar | 1g |
| Water | 20g |
| Marvie (trade mark) | 15g |
| Kiwi fruit | 20g |
| Pineapple | 30g |
| Apple | 30g |
| Marvie (trade mark) | 25g |

Example 8

### Warabi-mochi (Bracken starch pastry)

| | |
|---|---|
| Claimed product | 4g |
| Bracken powder | 20g |
| Marvie (trade mark) | 30g |
| Water | 70g |
| Soybean flour | 5g |

Example 9

### Hot cake

| | |
|---|---|
| Claimed product | 5g |
| Wheat flour | 30g |
| Egg | 10g |
| Baking powder | a little |
| Vanilla essence | a little |
| Vegetable oil | 2g |

Example 10

Oroshi-ae (Vegetables dressed with grated radish)

| | |
|---|---|
| Claimed product | 4g |
| Radish | 80g |
| Pholiota nameko | 30g |
| Green Welsh onion | 5g |
| Soy sauce | 4.8 m |

Example 11

Sujoyo-ae (Cucumber dressed with vinegar and soy)

| | |
|---|---|
| Claimed product | 4g |
| Agar-agar | 2g |
| Water | 40g |
| Egg | 20g |
| Cucumber | 30g |
| Soy sauce | 3.6 ml |

## Example 12

### Chinese salad

| | |
|---|---|
| Claimed product | 4g |
| Agar-agar | 2g |
| Water | 40g |
| Egg | 20g |
| Bean sprouts | 50g |
| Cucumber | 30g |
| Carrot | 20g |
| Raw <u>Shiitake</u> | 5g |
| Tomato | 10g |
| Sesami oil | 1g |
| Soy sauce | 9.6 ml |

## Example 13

### Salad dressed with sesami oil and vinegar

| | |
|---|---|
| Claimed product | 4g |
| Agar-agar | 2g |
| Water | 40g |
| Egg | 20g |
| Bean sprouts | 50g |
| Young rape greens | 20g |
| Carrot | 10g |
| Raw <u>Shiitake</u> | 10g |
| Tomato | 10g |
| Sesami oil | 2g |
| Soy sauce | 9.6 m |

Example 14

Sea food risotto

| | |
|---|---|
| Claimed product | 8g |
| Boiled rice | 80g |
| Asari with shell (Tapes philippinarum) | 50g |
| Shrimps | 60g |
| Tomato | 50g |
| Parsley | a little |
| Butter | 2g |
| Table salt | 2.5g |

Example 15

Ocimum and bean curd

| | |
|---|---|
| Claimed product | 5g |
| Silky bean curd | 100g |
| Agar-agar | 2g |
| Ohba (Perilla frutescens var. crispa | 1 leaf |
| Tomato | 15g |
| Soy sauce | 6 ml |
| Raw race ginger | a little |

Example 16

Mahbo-dofu (bean curd dish)

| | |
|---|---|
| Claimed product | 6g |
| Silky bean curd | 100g |
| Minced red pork | 30g |
| Onion | 40g |
| Bamboo shoot | 20g |
| Green Welsh onion | 5g |
| Dry Shiitake | 1g |
| Vegetable oil | 1g |
| Sesami oil | 1g |
| Cayenne pepper | a little |
| Soy sauce | 10.8 ml |

Example 17

Plain omelette

| | |
|---|---|
| Claimed product | 5g |
| Egg | 50g |
| White of an egg | 30g |
| Vegetable oil | 3g |
| Table salt | a little |
| Pepper | a little |

Example 18

Omelette

| | |
|---|---|
| Claimed product | 5g |
| Egg | 50g |

| | |
|---|---|
| Vegetable oil | 2g |
| Minced red beef | 30g |
| Onion | 40g |
| Carrot | 10g |
| Table salt | a little |
| Tomato ketchup | 5g |

Example 19

Corn salad

| | |
|---|---|
| Claimed product | 4g |
| Agar-agar | 2g |
| Corn | 10g |
| Cucumber | 30g |
| Carrot | 20g |
| Mayonnaise | 8g |
| Table salt | 0.3g |
| Lettuce | 15g |

Example 20

Hijiki (seaweed)

| | |
|---|---|
| Claimed product | 2g |
| Hijiki (seaweed) | 5g |
| Aburaage (fried bean curd) | 5g |
| Carrot | 10g |
| Sando-mame (snap beans) | 10g |
| Soy sauce | 4.8 ml |
| Marvie (trade mark | a little |

Example 21

**<u>Dried strips of radishes boiled</u>**

| | |
|---|---|
| Claimed product | 2g |
| Dried strips of radishes | 7g |
| Carrot | 10g |
| <u>Sando-mame</u> (snap beans) | 10g |
| Soy beans | 4.8 ml |
| Marvie (trade mark) | a little |

Example 22

**<u>Tomato cup</u>**

| | |
|---|---|
| Claimed product | 2g |
| Tomato | 100g |
| Cucumber | 15g |
| Mayonnaise | 5g |
| Table salt | a little |
| Pepper | a little |

Example 23

**<u>Vinegared dish (1)</u>**

| | |
|---|---|
| Claimed product | 3g |
| Raw <u>Wakame</u> (seaweed) | 5g |
| <u>Ohba</u> (<u>Perilla frutescens</u> var. <u>crispa</u>) | a little |
| Tomato | 40g |
| Table salt | a little |
| Vinegar | a little |
| Marvie (trade mark) | a little |

Example 24

### Vinegared dish (2)

| | |
|---|---|
| Claimed product | 3g |
| Mozuku (seaweed) | 50g |
| Cuttle fish | 20g |
| Ohba (perilla frutescens crispa) | a little |
| Soy sauce | 3.6 ml |
| Vinegar | a little |
| Marvie (trade Mark) | a little |

Example 25

### Vinegared dish (3)

| | |
|---|---|
| Claimed product | 4g |
| Agar-agar | 2g |
| Water | 40g |
| Crab (canned) | 20g |
| Cucumber | 40g |
| Ohba | a little |
| Table salt | a little |
| Vinegar | a little |
| Marvie (trade mark) | a little |

Example 26

### Chinese salad

| | |
|---|---|
| Claimed product | 4g |
| Agar-agar | 2g |
| Water | 40g |
| Pressed ham | 20g |
| Bean sprouts | 50g |
| Cucumber | 30g |
| Raw Shiitake | 5g |
| Sesami oil | 1g |
| Soy sauce | 4.8 ml |

Example 27

Chikuzenni (Chicken and vegetables cooked with soy)

| | |
|---|---|
| Claimed product | 2g |
| Chicken (thigh) | 30g |
| Burdock | 30g |
| Green peas | 3g |
| Konjak | 20g |
| Soy sauce | 4.8 ml |
| Marvie (trade mark) | a little |

Example 28

Soy beans boiled with soy

| | |
|---|---|
| Claimed product | 2g |
| Soy beans | 10g |
| Burdock | 30g |
| Carrot | 10g |

| | |
|---|---|
| Konjak | 30g |
| Konbu (seaweed) | a little |
| Green peas | 3g |
| Soy sauce | 4.8 ml |
| Marvie (trade mark) | a little |

Example 29

Chinese cabbage roll

| | |
|---|---|
| Chinese cabbage | 100g |
| Claimed product | 5g |
| Minced chicken | 60g |
| Onion | 40g |
| Carrot | 10g |
| Raw Shiitake | 10g |
| Table salt | a little |
| Pepper | a little |
| Usukuchi soy sauce | 6 ml |

Example 30

Cabbage roll

| | |
|---|---|
| Cabbage | 100g |
| Claimed product | 5g |
| Minced chicken | 60g |
| Carrot | 10g |
| Raw Shiitake | 10g |
| Table salt | a little |
| Pepper | a little |
| Usukuchi soy sauce | 6 ml |

Example 31

### Hamburg steak

| | |
|---|---|
| Claimed product | 5g |
| Minced red beef | 40g |
| Minced red pork | 40g |
| Onion | 50g |
| Bread crumb | 5g |
| Table salt | a little |
| Nutmeg powder | a little |
| Pepper | a little |
| Tomato ketchup | 10g |

Example 32

### Pressed chicken

| | |
|---|---|
| Claimed product | 5g |
| Minced chicken | 70g |
| Onion | 50g |
| Bread crumb | 5g |
| Miso | 10g |
| Table salt | a little |
| Pepper | a little |
| Poppy seeds | a little |

Example 33

### Shao-mai-like dish

| | |
|---|---|
| Claimed product | 5g |
| Minced chicken | 80g |
| Onion | 40g |
| Carrot | 15g |
| Table salt | a little |
| Pepper | a little |
| Green peas | 2g |
| Soy sauce | 6 ml |
| Vinegar | a little |
| Mustard | a little |

Example 34

### Shiitake filled with minced meat

| | |
|---|---|
| Raw Shiitake | 60g |
| Claimed product | 5g |
| Minced chicken | 40g |
| Bamboo shoot | 20g |
| Table salt | a little |
| Pepper | a little |
| Soy sauce | 6 ml |
| Vinegar | a little |
| Mustard | a little |
| Green pepper | 20g |
| Carrot | 30g |

Example 35

### Meat sauce

| | |
|---|---|
| Eggplant | 100g |
| Claimed product | 5g |
| Minced red beef | 60g |
| Onion | 40g |
| Carrot | 20g |
| Tomato | 60g |
| Raw Shiitake | 10g |
| Parsley | a little |
| Tomato ketchup | 20g |
| Worcester sauce | 3.6 g |
| Soy sauce | 2.4 ml |
| Table salt | a little |

Example 36

### Cup eggplant

| | |
|---|---|
| Eggplant | 100g |
| Claimed product | 5g |
| Minced chicken | 25g |
| Miso | 5g |
| White miso | 5g |
| Sesami seeds | 2g |

Example 37

### Umani (meat and eggplants boiled in soy)

| Claimed product | 3g |
| Pork (thigh) | 30g |
| Eggplants | 80g |
| Ginger root | a little |
| Soy sauce | 6 ml |
| Marvie (trade mark) | a little |

Example 38

### Peanuts-dressed dish

| Claimed product | 4g |
| Water | 40g |
| Agar-agar | 2g |
| Cucumber | 50g |
| Carrot | 10g |
| Raw Shiitake | 10g |
| Peanuts | 5g |
| Soy sauce | 6 ml |

Example 39

### Stuffed Spanish paprika

| Spanish paprika | 45g |
| Claimed product | 4.5g |
| Minced chicken | 25g |
| Onion | 20g |
| Carrot | 10g |
| Raw Shiitake | 5g |
| Table salt | a little |
| Pepper | a little |
| Tomato ketchup | 10g |

Example 40

### Meat and vegetables boiled in soy

| | |
|---|---|
| Dried bean curd | 15g |
| Claimed product | 3g |
| Minced chicken | 20g |
| Carrot | 20g |
| Raw shiitake | 10g |
| Snap beans | 10g |
| Soy sauce | 3 ml |
| Marvie (trade mark) | a little |
| Table salt | a little |

Example 41

### Dish with brown sauce

| | |
|---|---|
| Claimed product | 5g |
| Minced red beef | 40g |
| Minced red pork | 40g |
| Onion | 50g |
| Egg | 10g |
| Table salt | a little |
| Pepper | a little |
| Wheat flour | 3g |
| Vegetable oil | 3g |
| Tomato ketchup | 20g |
| Worcester sauce | 6g |

Example 42

### Omelette rice

| | |
|---|---|
| Boiled rice | 80g |
| Egg | 50g |
| Vegetable oil | 2g |
| Claimed product | 8g |
| Chicken (thigh) | 30g |
| Onion | 30g |
| Carrot | 10g |
| Raw Shiitake | 5g |
| Spanish paprika | 5g |
| Vegetable oil | 3g |
| Tomato ketchup | 5g |
| Table salt | a little |

Example 43

### Chinese fried rice

| | |
|---|---|
| Claimed product | 8g |
| Boiled rice | 80g |
| Minced red pork | 30g |
| Onion | 30g |
| Carrot | 10g |
| Corn | 10g |
| Green peas | 3g |
| Vegetable oil | 2g |
| Table salt | a little |
| Pepper | a little |
| Curry powder | a little |
| Soy sauce | 3.6 ml |

Example 44

Chakin-zushi (Vinegared rice wrapped with omelette)

| | |
|---|---|
| Egg | 50g |
| Vegetable oil | 2g |
| Claimed product | 8g |
| Boiled rice | 80g |
| Table salt | a little |
| Vinegar | a little |
| Marvie (trade mark) | a little |
| Dry Shiitake | 1g |
| Dried gourd shavings | 1g |
| Soy sauce | a little |
| Mitsuba | 10g |

Example 45

Porridge of rice and vegetables

| | |
|---|---|
| Claimed product | 8g |
| Boiled rice | 80g |
| Egg | 40g |
| Greens | 30g |
| Chinese cabbage | 30g |
| Carrot | 15g |
| Soy sauce | 15 ml |
| Katsuodashi (bonito stock) | a little |

Example 46

### Risotto

| | |
|---|---|
| Claimed product | 8g |
| Boiled rice | 80g |
| Chicken (breast) | 40g |
| Spinach | 30g |
| Carrot | 20g |
| Shimeji | 20g |
| Table salt | a little |
| Pepper | a little |
| Chicken soup | a little |

Example 47

### Iri-dofu (Bean curd boiled dry and seasoned)

| | |
|---|---|
| Claimed product | 5g |
| Silky bean curd | 130g |
| Minced red pork | 30g |
| Onion | 40g |
| Bamboo shoot | 20g |
| Carrot | 10g |
| Green Welsh onion | 5g |
| Dry Shiitake | 1g |
| Miso | 10g |
| Vegetable oil | 2g |
| Soy sauce | 15.6 ml |

Example 48

Okonomiyaki (Pancake with meat and vegetables)

| | |
|---|---|
| Claimed product | 8g |
| Wheat flour | 30g |
| Egg | 30g |
| Minced red pork | 30g |
| Cabbage | 50g |
| Carrot | 5g |
| Raw Shiitake | 5g |
| Vegetable oil | 3g |
| Dried bonito flakes | 2g |
| Tonkatsu sauce (thick sauce) | 15g |

Example 49

Sarasayaki

| | |
|---|---|
| Claimed product | 5g |
| Egg | 50g |
| White of egg | 30g |
| Minced chicken | 20g |
| Onion | 30g |
| Carrot | 10g |
| French beans | 10g |
| Dry Shiitake | 1g |
| Vegetable oil | 3g |
| Table salt | a little |
| Pepper | a little |

## Example 50

### Yoghourt

| | |
|---|---|
| Claimed product | 10g |
| Yoghourt | 160g |
| Aspartane | 0.034g |

## Example 51

### Dry yoghourt (1)

The above yoghourt (50) was freeze-dried. The dry yoghourt is eaten after adding 140 ml of water thereto.

## Example 52

### Dry yoghourt (2)

| | |
|---|---|
| Claimed product | 10g |
| Water | 140 ml |

These two components were mixed and allowed to stand and then freeze-dried. The resultant is called 'the claimed dry product'.

| | |
|---|---|
| Claimed dry product | 10g |
| Dry yoghourt powder | 20g |
| Aspartane | 0.034g |

The dry yoghourt (2) is eaten after adding 140 ml of water thereto.

## Example 53

### Dry yoghourt (3)

| | |
|---|---|
| Claimed product | 10g |
| Dry yoghourt powder | 20g |
| Aspartane | 0.034g |

The dry yoghourt (3) is eaten after adding 140 ml of water thereto.

The following are application examples of food compositions and dieting agents in accordance with the invention.

### Example 54 (Effect on in-patients having simple obesity)

To 14 female patients aged 35-70 who were admitted to the hospital with a diagnosis of simple obesity and showing an obesity index (Brocar index) of 120 or more were fed food compositions of the invention with other foods at meals, to give a daily dosage of Ocimum fruit of 10g a day. The Ocimum fruit was added to foods

EP 0 333 342 B1

according to the above various cooking examples to be fed to the patients at meals.

Each patient had the average intake of about 800Kcal a day whilst taking the food composition of the present invention.

The average term of taking the food composition was about 4 months (120 ± 94 days).

The patients weighed 62.3kg each on average before taking the food composition of the present invention but decreased to 54.2kg each after the period of administration, a percentage decrease of about 13%. Also, the obesity index of 133 decreased to 116 in a percentage decrease of about 13% to show a low rate of p<0.005 with a sufficiently significant difference.

The average value of triglycerides in the blood serum of the patients decreased from 166 to 11 mg/dl, a marked decrease of about 33%.

The patients were questioned as to the taste of the dishes of the present invention. 92% of the patients said 'good' and 8% 'bad'. It was found that the food composition of the invention exerted a good influence on the taste of most foods.

The patients were also questioned as to the feeling of fullness given by the foods including the food composition of the invention, and 96% of the patients answered yes. It was said that obese patients usually had to have an intake of about 2000Kcal each to have a feeling of fullness after a meal. However, it was found that the intake of only about 800 Kcal whilst administering the food composition of the invention gave a feeling of fullness to the patients.

Example 55 (Effect on Zucker's obese rats 'fatty')

Male obese rats (Fatty) aged 8 weeks were divided into two groups. One was control group consisting of 7 rats and the other was an administration group of 6 rats. The control group were fed with Rat Chow MB-2 (made by Funabashi Farm Co., Ltd.) and the administration group was fed with a mixture of the Rat Chow MB-2 and the fruit of Ocimum canum (containing a final concentration of 5% Ocimum fruit for 28 days ad libitum.

Each rat of the administration group was fed with the dieting agent of the invention in an amount of 4.89 g/kg/day, calculated from the average intake of feed.

Each rat of the administration group showed a loss of 3% in weight in the end when compared with the control group. Also, each rat of the administration group had an average decrease of 7% in the intake to show a significant difference with a peril rate of p<0.02 when compared with the control. This reveals that the rats of the administration group had a feeling of fullness.

Also, when the triglycerides in the blood serum of both groups were measured, the administration group showed an average decrease of 24% each. With regard to the total value of cholesterol in the blood serum, the administration group showed an average decrease of 13% each, and as to the amount of glucose in the blood serum, the administration group had an average decrease of 23% each, to show a significant difference with a peril rate of p<0.05. The inevitable accumulation of lipid in the blood due to obesity was markedly improved.

Example 56 (Use of Ocimum fruit in yoghourt

(1) To 10g of Ocimum fruit were added 20g of dry yoghourt powder (Minami Dairy Fermentation Powder M-Y 500 made by Minami Nippon Rakuno Kyodo Co., Ltd.) and 2g of Pat Sweet (trade mark, sweetening containing 1.7% of aspartane made by Ajinomoto Co., Inc.), and the mixture was uniformly stirred. The thus obtained dry powder mixture was watered with 140ml of water and allowed to stand for about 30 min. before eating.

(2) To 10g of Ocimum fruit were added 160g of Meiji Bulgaria Yoghourt LB51 (made by Meiji Milk Products Co., Ltd.) and 2g of Healsweet (trade mark, sweetening containing 22% of sugar-changed Stevia; made by Suehiro Enzyme Research Institute) with stirring. Stirring was carried out to uniformly disperse the Ocimum fruit and Healsweet in the yoghourt. After the completion of stirring, the yoghourt was allowed to stand for about 30 min. and then eaten as food.

(3) To 10g of Ocimum fruit were added 160g of Meiji Bulgaria Yoghourt LB51 and 2g of Pal Sweet, 14 drops of natural red colouring matter (made from Benikoji (red Aspergillus) by Asaoka Perfume Co., Ltd.), and 7 drops of strawberry essence (made by Asaoka Perfume Co., Ltd.). The mixture was uniformly stirred and then allowed to stand for about 30 min. to obtain a strawberry-flavoured yoghourt.

(4) To 10g of Ocimum fruit were added 160g of Meiji Bulgaria Yoghourt LB51, 2g of Pal Sweet (trade mark) and 4.6g of Van Houten cocoa previously dissolved in 14ml of warm water (made by Kataoka Bussan Co., Ltd.; containing 22-24% of cocoa butter). The mixture was uniformly stirred and then allowed to stand for about 30 min. to obtain a chocolate-flavoured yoghourt.

(5) To 10g of Ocimum fruit were added 160g of Meiji Bulgaria yoghourt LB51, 2g of Pal Sweet (trade mark), and 2g of powdered tea. The mixture was uniformly stirred and then allowed to stand for about 30 minutes to obtain a powdered tea-flavoured yoghourt.

(6) To 10g of Ocimum fruit were added 160g of Meiji Bulgaria yoghourt LB51, 2g of Pal Sweet, and 8g of Blueberry Sauce (made by Morozofu Co., Ltd.). The mixture was uniformly stirred and then allowed to stand for about 30 min. to obtain a blueberry-flavoured yoghourt.

The inventors have studied the compositions of yoghourt prepared in accordance with the present invention to obtain the above 6 most preferable examples.

The following facts were clarified as to the yoghourt of the present invention in the experiments conducted by the inventors.

(1) The addition of only the Ocimum fruit of the present invention to yoghourt improved the taste, sourness, and feeling thereof.

(2) The further addition of sweetening to the mixture of the Ocimum fruit and yoghourt improved the taste and feeling, and particularly that of 0.5g of sweetening, Pal Sweet (trade mark) to 40g of yoghourt was most preferable.

(3) The good taste of Ocimum fruit had no difference even when the amount of Pal Sweet was changed up to 2g per 40g of yoghourt.

(4) The addition of water lowered the sweetness and increased the sourness but improved the feeling.

(5) As to the taste, the amount of the Ocimum fruit can be increased up to 3g per 40g of yoghourt, and further the addition of water made the taste better.

## Claims

### Claim for the following Contracting Sates : AT, BE, CH, DE, FR, GB, IT, LU, NL, SE

1.  The use of the fruit of a plant of the genus Ocimum in the preparation of an obesity control agent.

### Claims for the following Contracting States : ES, GR

1.  A process for the preparation of an obesity control agent in which there is used, as active ingredient, the fruit of a plant of the genus Ocimum.

2.  The use of the fruit of a plant of the genus Ocimum in the preparation of an obesity control agent.

## Patentansprüche

### Patentanspruch für folgende Vertragsstaaten : AT, BE, CH, DE, FR, GB, IT, LU, NL, SE

1.  Die Verwendung der Frucht einer Pflanze der Gattung Ocimum bei der Zubereitung eines Fettleibigkeitssteuermittels.

### Patentansprüche für folgende Vertragsstaaten : ES, GR

1.  Ein Verfahren für die Zubereitung eines Fettleibigkeitssteuermittels, bei dem als aktive Ingredienz die Frucht einer Pflanze der Gattung Ocimum verwendet wird.

2.  Die Verwendung der Frucht einer Pfanze der Gattung Ocimum bei der Zubereitung eines Fettleibigkeitssteuermittels.

## Revendications

### Revendication pour les Etats contractants suivants : AT, BE, CH, DE, FR, GB, IT, LU, NL, SE

1.  Utilisation du fruit d'une plante du genre Ocimum pour la préparation d'une composition contre l'obésité.

**EP 0 333 342 B1**

**Revendications pour les Etats contractants suivants : ES, GR**

1.  Procédé de préparation d'une composition contre l'obésité, dans lequel on utilise, comme substance active, le fruit d'une plante du genre <u>Ocimum</u>.

2.  Utilisation du fruit d'une plante du genre <u>Ocimum</u> pour la préparation d'une composition contre l'obésité.

27